# EUROPEAN PATENT APPLICATION

(11) **EP 1 645 303 A2**
(43) Date of publication of application: **12.04.2006**
(21) Application number: 05256130.5
(22) Date of filing: 30.09.2005
(51) Int. Cl.: A61M 16/00, A61M 16/06

(54) **Fluid flow sensor**

(30) Priority: 02.10.2004 GB 0421943
(71) Applicant: Oxymon Limited, West Sussex, BN6 8HD (GB)
(72) Inventor: Jones, David Arthur, West Sussex, BN6 8HD (GB); Poncelet, Noël, Hassocks West Sussex, BN6 8HD (GB)
(74) Representative: Feakins, Graham Allan

(57) **Abstract**

A fluid flow sensor for a device, such as breathing apparatus (1) in the form of a cannula pipe or face mask, the sensor having means (7A) for monitoring and indicating the pressure or absence of a fluid flow in a conduit (3) for supplying fluid from a fluid supply (2) to the device. A means is provided responsive to back pressure in the conduit (3) to actuate an indicator (5) of the sensor to alert a use of the device of the presence and/or absence of the fluid flow.

## Description

This invention relates to a fluid flow sensor for a device and in particular to a gas flow sensor for breathing apparatus.

Users of breathing apparatus supplying oxygen from a bottle, for example, can experience problems, especially if no indication is given that the oxygen supply from the bottle is running low or actually runs out, thereby leaving the user in a vulnerable situation of having no oxygen to breathe.

A similar situation can arise when the oxygen gas tap is open but the bottle itself is not in use, thereby wasting oxygen and potentially leaving the user with an empty oxygen bottle. There is also a fire risk if oxygen continues to leak from the bottle creating a potentially combustible atmosphere environment.

According to the present invention, there is provided a fluid flow sensor for a device, the sensor comprising means for monitoring and indicating the presence or absence of a fluid flow in a conduit for supplying fluid from a fluid supply to the device, including means responsive to back pressure in the conduit to actuate an indicator of the sensor to alert a user of the device of the presence and/or absence of the fluid flow, the back pressure being caused by a fluid flow limiter provided downstream of said fluid supply and the sensor being located to detect such back pressure.

The invention is particularly applicable where the device is in the form of breathing apparatus such as a cannula pipe or face mask supplied by a pipe (conduit) with oxygen from an oxygen bottle with the sensor acting to sense the flow of gas to the apparatus. The flow limiter can simply be, for example, the cannula or face mask itself or the tubing supplying the apparatus.

The sensor can include an audible alarm to warn the user as and when a hazardous situation is likely to occur.

The sensor can include a visual indicator and/or an audible alarm to indicate the current state of fluid monitoring.

The sensor may also include a "silence button" or other means in order to suppress temporarily a warning alarm.

The sensor may be battery-operated.

The sensor may be provided with an alarm to warn the user that the battery supply is running low, which therefore may result in the imminent failure of the sensor to monitor the fluid flow.

The sensor may have means to configure a timeout period in order to suit a medical working environment, for example, as well as a domestic user environment.

Preferably, the sensor is a micro-processor-based apparatus.

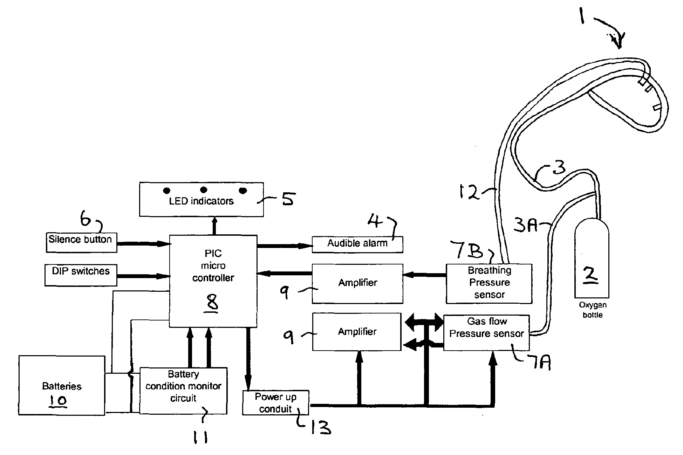

For a better understanding of the invention and to show how the same may be carried into effect, reference will now be made, by way of example, to the accompanying drawing, in which the single figure shows a fluid flow sensor for a device, the device being breathing apparatus 1 in this case fed by oxygen supplied from a bottle 2.

The sensor works on the principle that a cannula pipe 3 feeding oxygen from the bottle to the user will produce back pressure that can be detected using a fluid flow limiter (not shown) located in the pipe 3 downstream of the branch 3A leading to the pressure sensor (to be described later), and used to identify when the gas tap (not shown) is on. Knowing that gas is flowing allows the situation to be monitored and potentially hazardous situations highlighted to the user as necessary.

As can be seen, the sensor includes an audible alarm 4 to warn the user as and when a hazardous situation is likely to occur. Visual indicators 5 and alarms indicate the current state of gas monitoring. There is a silence button 6 to silence a warning alarm temporarily.

The sensor is arranged always to be in its operative, on mode but is constructed such that it is only of low power consumption, so that the batteries will last at least the usage period of the gas bottle. However, a "low battery" alarm is provided in order to warn the user that the sensor may cease monitoring imminently.

In the "medical" mode, the monitoring period before warning the user that the gas tap may have been left on is very short, e.g. in the region of five minutes. When the sensor is in its "domestic" mode, the period of time before warning the user can be arranged to be of the order of one hour. This is particularly because a domestic user may use the bottle for longer periods than, say in a hospital environment, so that the alarms would become quite irritating if they repeated every five minutes.

Pressure detection is achieved using piezo-electric pressure sensing devices 7a and 7B. This provides a differential voltage output that is fed to a microcontroller 8 via amplifiers 9. Pressures lower than 1 pound per square inch - 1bf/in² (6.895 Kn/m²) can be detected, whilst the sensor can sustain its function during a period of over-pressure of up to 45 1bf/in² (310.28 Kn/m²).

Once gas flow has been detected, a green LED will flash periodically to indicate that the flow of gas has been detected. Once a period D1 has elapsed, an orange LED will flash periodically, along with the green LED. This indicates that the gas tap has been on but not for an excessive period of time. The period T1 is two and a half minutes when the sensor is in "medical" mode and half an hour when in "domestic" mode. When a further T1 period has elapsed, an audible warning will sound and a red LED will also flash periodically. It is now deemed a suitable time to warn the user that the gas tap may have been left on inadvertently.

Once the audible warning has sounded, it may be silenced for a period of T2 by pressing the silence button 6. This allows the user to be freed from continuous alarms, while at the same time allowing the sensor to continue monitoring that the gas is flowing. When the period T2 has expired, the alarms can be silenced again any number of times until either the gas tap is turned off or the bottle runs out of oxygen.

Once gas flow is detected, the pressure is continuously monitored. Under normal conditions, the pressure will drop sharply when the gas tap is turned off after use. When this occurs, the sensor will reset itself to an idle state and will await the next time gas pressure is detected.

If, however, the gas pressure slowly begins to drop, this will indicate that the gas bottle is becoming empty. Under these circumstances, the sensor will sound a distinct alarm to warn the user of the imminent loss of oxygen.

It will be appreciated that pipe blockages or restricted oxygen flows can also be detected because this will cause the back pressure to rise to an abnormally high level. Should a high back pressure be detected this will be signalled to the user by an audible alarm to warn them against the potentially life threatening situation of insufficient oxygen being delivered.

The condition of the or each battery 10 is continuously monitored using a battery condition monitoring circuit 11, such that, if a low battery condition is detected, a distinctive alarm is sounded to alert the user. This alarm will continue periodically until either the batteries are replaced or the batteries finally become too low to work the sensor.

By using a second pipe 12 running alongside the oxygen delivery cannula pipe 3, the user's breathing can be detected. At one end the pipe 12 is connected to a second pressure sensor 7B, and the other end of the pipe 12 ends flush with cannula nasal pipes of the breathing apparatus 1. As the user breathes, a variation in pressure will result within the second pipe 12 that can be detected. These variations will be analysed and checked to ensure that they fall between predefined limits. Should they fall outside the expected levels and periodicities, an audible alarm will be raised.

Breathing detection is not only a safety feature, but is also a method of improving compliance with treatment. It is common for patients to have the mask or cannula poorly positioned thus reducing the effectiveness of the treatment. By monitoring breathing, improvements in safety and effectiveness of treatment can be achieved. Breathing detection may also aid recognition of situations where the oxygen flow has been left on unintentionally.

On power-up using a power-up circuit 13, the sensor will self-calibrate by taking a number of readings from the pressure sensor and use these as a "zero gas flow" setting.

When in the idle state, the sensor continuously auto-zeros.

Provision is also made for data storage of user actions with ability to download the data for analysis.

## Claims

1. A fluid flow sensor for a device, the sensor comprising means (7A, 7B) for monitoring and indicating the presence or absence of a fluid flow in a conduit (3) for supplying fluid from a fluid supply to the device, **characterised by** means (7A) responsive to back pressure in the conduit (3) to actuate an indicator (5) of the sensor to alert a user of the device of the presence and/or absence of the fluid flow, the back pressure being caused by a fluid flow limiter provided downstream of said fluid supply (2) and the sensor being located to detect such back pressure.

2. A sensor according to claim 1, and including an audible alarm (4) to warn the user as and when a hazardous situation is likely to occur.

3. A sensor according to claim 1 or 2, and including a visual indicator (5) to indicate the current state of fluid monitoring.

4. A sensor according to claim 1, 2 or 3, and including an audible alarm (4) to indicate the current state of fluid monitoring.

5. A sensor according to any one of the preceding claims, and including means to suppress temporarily a warning alarm.

6. A sensor according to any one of the preceding claims, and being battery-operated.

7. A sensor according to claim 6, being provided with a low-battery alarm (11).

8. A sensor according to any one of the preceding claims, and having means to configure a time-out period in order to suit a medical working environment and/or a domestic user environment.

9. A sensor according to any one of the preceding claims, and being a micro-processor-based apparatus (8).

10. A sensor according to any one of the preceding claims, and including a piezo-electric pressure sensing device (7A).

11. A sensor according to any one of the preceding claims, and being adapted to sense the flow of gas to a breathing apparatus such as a cannula pipe or face mask (1).

12. Breathing apparatus including a sensor according to any one of the preceding claims.

13. Breathing apparatus according to claim 12, and being in the form of a cannula pipe or face mask.

14. Breathing apparatus according to claim 13, wherein the flow limiter is provided by the cannula pipe or face mask (1) itself and/or the conduit (3) supplying the apparatus with gas.

15. Breathing apparatus according to claim 13 or 14, wherein said sensor includes a gas flow pressure sensor (7A) and a breathing pressure sensor (7B), the latter being provided with its own pipe (12) leading from the breathing apparatus (1).
